# EUROPÄISCHE PATENTSCHRIFT

(11) **EP 2 854 720 B1**
(45) Veröffentlichungstag und Bekanntmachung des Hinweises auf die Patenterteilung: **29.03.2017**
(21) Anmeldenummer: 13724605.4
(22) Anmeldetag: 24.05.2013
(51) Int. Cl.: A61F 5/01

(54) **ORTHOPÄDISCHE VORRICHTUNG ZUR BEGRENZUNG DER BEWEGUNG EINES ZWISCHEN EINEM ERSTEN UND ZWEITEN KÖRPERBEREICH ANGEORDNETEN GELENKS**
ORTHOPAEDIC DEVICE FOR LIMITING THE MOVEMENT OF A JOINT ARRANGED BETWEEN A FIRST AND A SECOND REGION OF A BODY
DISPOSITIF ORTHOPÉDIQUE PERMETTANT DE LIMITER LE MOUVEMENT D'UNE ARTICULATION PLACÉE ENTRE UNE PREMIÈRE ET UNE DEUXIÈME PARTIE DU CORPS

(30) Priorität: 25.05.2012 DE 102012011433
(43) Veröffentlichungstag der Anmeldung: 08.04.2015
(73) Patentinhaber: Betterguards Technology GmbH, 10625 Berlin (DE)
(72) Erfinder: Bichler, Vinzenz, 10777 Berlin (DE)
(74) Vertreter: Nordmeyer, Philipp Werner
(86) Internationale Anmeldenummer: PCT/EP2013/060771
(87) Internationale Veröffentlichungsnummer: WO 2013/174989

(56) Entgegenhaltungen:
- US-A- 4 471 538
- US-A- 5 712 011
- US-A1- 2007 107 778
- US-A1- 2012 089 065
- US-B1- 7 402 147

## Beschreibung

### Technisches Gebiet

Die vorliegende Erfindung betrifft eine orthopädische Vorrichtung zur Begrenzung der Bewegung eines zwischen einem ersten und zweiten Körperbereich angeordneten Gelenks, umfassend zumindest eine an dem ersten Körperbereich fixierbare Aufnahme und einen an dem zweiten Körperbereich fixierbaren und relativ zu der Aufnahme bewegbaren Auszugskörper.

### Stand der Technik

Schädigungen im Bereich der am Bewegungszyklus beteiligten Gelenke zählen zu einem der wichtigsten Unfallschwerpunkte. Dabei sind besonders die so genannten "Stolper-, Rutsch- und Sturzunfälle" (SRS-Unfälle) hervorzuheben, welche als Verletzungsursache bei Wege-, Arbeits- und Sportunfällen am häufigsten vorkommen. Diese Unfälle können einzeln oder in Kombination zu erheblichen Verletzungen führen. Überwiegend betroffen sind vor allem größere Gelenke, wie das obere und untere Sprunggelenk (articulatio talocuralis und articulatio talotarsalis), das Handgelenk (articulatio manus) sowie das Kniegelenk (articulatio genus). Dabei ist eine Verletzungstendenz in diesem Bereich auch sportmedizinisch zu verzeichnen.

Durch die SRS-Unfälle kommt es häufig zu einer gewaltsamen Überdehnung des Halteapparats des Gelenks (Gelenkkapsel, Bänder, Sehnen, Knochen) über den physiologisch maximal möglichen Winkel. Diese Überdehnung ist auch bekannt unter den Begriffen Distorsion beziehungsweise Verstauchung. Da dies beim Sprung- und Handgelenk oft in Supinationsrichtung auftritt, spricht man in diesem Fall von einem so genannten Supinationstrauma. Dabei handelt es sich um ein Synonym für eine Verletzung, bei der eine über den physiologischen Bewegungsspielraum erfolgte Supination des Fußes zusammen mit einer Plantarflexion zu einer Schädigung der lateralen Knochenstrukturen und des lateralen Kolateralbandapparates führt. Supinationstraumata äußern sich zunächst in starken Schmerzen, die meist mit einer Schwellung der Gliedmaße und der Einwanderung eines Hämatoms (im Fall von ruptierten Bändern) verbunden sind. Werden solche Begleiterscheinungen nicht erkannt und medizinisch ausreichend versorgt, können Spätfolgen, wie beispielsweise eine chronische Instabilität des Gelenks oder eine Gelenkarthrose, eintreten.

Ein sehr wichtiger Faktor für den positiven Heilungsverlauf eines Supinationstraumas ist die Stabilisierung der Gelenke des Patienten, zur Verletzungsprävention oder während der prä- oder postoperativen Versorgung. Dabei wird die Stabilisierung der Gelenke in der Regel über Tapeverbände, Funktionssicherungsorthesen, Stabilisierungsorthesen, Bandagen und/oder stabile Schuhe mit variabler Schafthöhe und optionalen Verstärkungselementen erreicht.

Unter Orthese wird im Allgemeinen und in dieser Anmeldung ein funktionssicherndes, körperumschließendes oder körperanliegendes Hilfsmittel verstanden, das von seiner physikalischen/mechanischen Leistung konstruktiv stabilisiert, immobilisiert, mobilisiert, entlastet, korrigiert, retiniert, fixiert, redressiert und/oder ausgefallene Körperfunktionen ersetzt.

Darüber hinaus können Orthesen aus körperteilumschließenden Schäften bestehen, die geschlossen oder gefenstert als Rahmenschäfte gearbeitet sind. Dabei werden die Schaftsysteme mittels Verschlüssen (beispielsweise Klettverschlüssen) am Körper fixiert. Ferner können die Schaftsysteme gelenkübergreifender Orthesen entsprechend den funktionellen Anforderungen mittels uni- oder bilateraler Schienensysteme miteinander verbunden werden. Des Weiteren kann zwischen statischen Orthesen zur Fixierung, Korrektur und Stützung eines oder mehrerer Gelenke und dynamischen Orthesen zur Aufbringung einer konstanten Kraft auf ein bewegliches Körperteil differenziert werden.

Zum Beispiel zeigt die DE 10 2005 002 834 B4 eine orthopädische Vorrichtung zur Stabilisierung, Entlastung und Führung des Fußes und der Zehen bei Lähmung der Dorsalex Tensoren von Fuß oder Zehen. Dabei umfasst die Vorrichtung eine Einrichtung zur Halterung am Unterschenkel, an welcher frontwärts und in Richtung des Fußes weisend eine vollständig oder abschnittsweise flexible Schiene reversibel oder irreversibel anbringbar ist.

Die US 5 472 412 zeigt eine gelenkige Knieorthese, welche ein Armpaar, welches durch entsprechende Schwenkstifte schwenkbar verbunden ist, und Zahnsegmente, welche durch einen Satz von Nabenplatten mit Löchern zur Aufnahme verstellbarer Anschlagbolzen definiert sind, umfasst. Dabei werden die Arme durch flexible und lösbare Bänder an das Bein einer Person angebracht, so dass sich ein Gelenk zwischen den beiden Armen befindet. Dabei umfasst einer der Arme eine hydraulische Steuereinheit, welche über einen Satz rotierbarer Ventile verfügt, mittels welcher der Fluss des hydraulischen Fluides durch die Ventile und zwischen zwei in der Steuereinheit angeordneten Kolbenkammern gesteuert werden kann. Auf diese Weise lässt sich ein Widerstand gegen Beugung und Streckung der Orthese und des Beines manuell einstellen.

Unter Bandagen werden im Allgemeinen und in der vorliegenden Anmeldung körperteilumschließende oder körperteilanliegende konfektionierte Hilfsmittel mit komprimierender und/oder funktionssichernder Wirkung verstanden. Dabei kommen in der Regel weiche, elastische textile Materialien zum Einsatz.

Bandagen können dazu dienen, Gelenke und Knochen vor einer Überbeanspruchung zu schützen oder aber akute oder chronische Weichteilerkrankungen zu behandeln.

In der EP 0 600 218 B1 wird eine Bandage für das Kniegelenk aus elastischem Bandagenstoff in Schlauchform mit einem vorderen Bandagenteil und einem rückwärtigen Bandagenteil und mit mindestens einem eingearbeiteten, längsverlaufenden Federstab gezeigt.

Unter einem Tapeverband wird im Allgemeinen und in der vorliegenden Anmeldung ein Verband zum Stabilisieren, Stützen und Halten von Extremitäten, Schutz vor weiteren Verletzungen, Reduzieren ungewollter Gelenkbewegungen sowie Unterstützen des Heilungsprozesses verstanden. Tapeverbände kommen entsprechend zur Profilaxe, Erstversorgung, Therapie und Rehabilitation zum Einsatz.

Orthesen weisen in der Regel eine komplexe Bauweise auf, welche meist mit einer komplizierten Anbringung am Körper beziehungsweise einer komplizierten Einstellung einhergeht. Darüber hinaus können Orthesen einen hohen Materialeinsatz erfordern, was nicht selten zu einer sperrigen Bauweise der Orthesen führt. Die Tatsache, dass der Widerstand der Orthesen in der Regel manuell eingestellt werden muss, ist als weiterer Nachteil anzusehen.

Bandagen weisen allgemein eine eingeschränkte Stützwirkung auf. Darüber hinaus ist das Tragen von Bandagen oft mit der Bildung von Druckstellen verbunden.

Nachteilig an Tapeverbänden ist, dass deren Stabilisierungswirkung nicht lange anhält. In der Regel liegt diese nach den ersten 10 Minuten lediglich bei 50%.

Die US 5,712,011 A zeigt eine gegen Zug widerstandsfähige Verbindung.

### Darstellung der Erfindung

Ausgehend von dem bekannten Stand der Technik ist es Aufgabe der vorliegenden Erfindung, eine orthopädische Vorrichtung zur Begrenzung der Bewegung eines zwischen einem ersten und einem zweiten Körperbereich angeordneten Gelenks, umfassend zumindest eine an dem ersten Körperbereich fixierbare Aufnahme und einen an dem zweiten Körperbereich fixierbaren und relativ zu der Aufnahme bewegbaren Auszugskörper, anzugeben, welche die Begrenzung der Bewegung verbessert.

Diese Aufgabe wird mittels einer Vorrichtung mit den Merkmalen des Anspruchs 1 gelöst. Vorteilhafte Ausgestaltungen ergeben sich aus den Unteransprüchen.

Entsprechend wird eine orthopädische Vorrichtung zur Begrenzung der Bewegung eines zwischen einem ersten und einem zweiten Körperbereich angeordneten Gelenks, umfassend zumindest eine an dem ersten Körperbereich fixierbare Aufnahme und einen an dem zweiten Körperbereich fixierbaren und relativ zu der Aufnahme bewegbaren Auszugskörper, angegeben. Erfindungsgemäß ist dabei zwischen der Aufnahme und dem Auszugskörper ein mit einem dilatanten Fluid gefülltes Volumen vorgesehen.

Dadurch kann für den Fall, dass das Gelenk eine hohe Krafteinwirkung oder eine ruckartige Bewegung erfährt, dieser entgegengewirkt werden, indem das dilatante Fluid die Beweglichkeit des Auszugskörpers relativ zur Aufnahme einschränkt. Hingegen erlaubt das dilatante Fluid bei langsamen, kraftarmen Gelenkbewegungen eine nahezu uneingeschränkte Beweglichkeit des Auszugskörpers relativ zu der Aufnahme.

Auf diese Weise können unerwünschte Bewegungen unterbunden werden. Beispielsweise können schnelle beziehungsweise ruckartige Bewegungen, welche sich negativ auf einen Heilungsprozess auswirken können, vermieden werden. Hinzu kommt, dass durch die Einschränkung der Bewegung auch ein Überstrecken beziehungsweise Überdehnen des jeweiligen Gelenks verhindert werden kann. Darüber hinaus kann die orthopädische Vorrichtung eine Verlängerung der Muskelreaktionszeit ermöglichen.

Das Gelenk beziehungsweise die entsprechenden Körperteile können auf diese Weise in Abhängigkeit von der einwirkenden Kraft gestützt und fixiert werden.

Durch die adaptive Bewegungsbegrenzung ergibt sich für die orthopädische Vorrichtung ein breites Anwendungsspektrum. So kann die orthopädische Vorrichtung beispielsweise sowohl an Orthesen und Protektoren, als auch an Bandagen angebracht werden. Darüber hinaus kommen auch Gebiete wie Sportausrüstung, Prothesen, Schutzkleidung und dergleichen in Betracht. Insbesondere kann die orthopädische Vorrichtung auch in Schuhen, Fitnessbandagen und/oder Arbeitshilfsmitteln iwe zum Beispiel wie zum Beispiel Handschuhen integriert werden.

Somit kann die orthopädische Vorrichtung sowohl für therapeutische als auch für prophylaktische Zwecke eingesetzt werden. Im Rahmen eines therapeutischen Einsatzes kann die Vorrichtung derart ausgeführt sein, dass Teilbelastungen des entsprechenden Körperteils zugelassen werde, was sich positiv auf den Heilungsverlauf auswirken kann.

Für eine adaptive Bewegungsbegrenzung kommen dilatante Materialien, wie beispielsweise Copolymerdispersionen oder -fluide, welche bei dem Auftreten von hohen Scherkräften und einer damit verbundenen hohen Schwergeschwindigkeit eine höhere Viskosität aufweisen, zum Einsatz. Je größer die aufgebrachte Scherung ist, desto viskoser beziehungsweise zähflüssiger verhält sich das Fluid. Entsprechend werden dilatanten Fluiden auch scherverfestigende beziehungsweise scherverdickende Eigenschaften zugeschrieben.

Unter dilatantem Fluid sind im Allgemeinen und insbesondere in der vorliegenden Anmeldung Copolymerdispersionen zu verstehen, wie sie beispielsweise in der DE 30 25 562 A1, der DE 34 33 085 A1 und der DE 39 17 456 A1 gezeigt sind. Die Dispersionen setzen sich beispielsweise aus Emulsionscopolymerisaten und Metallsalzen zusammen. Die Emulsionscopolymerisate können beispielsweise aus
1 - 10 Gew.-% monoolefinisch ungesättigten Mono- und/oder Dicarponsäuren, wie Acryl-, Methacryl-, Malein- und/oder Fumarsäure,
99 - 90 Gew.-% andere olefinisch ungesättigte Monomere, wie Styrol, C1-C6-Alkylacrylate, wie Methylmethacrylat, und
5 - 30 Gew.-% eines Carponsäurealylester-Monomers mit zwei oder mehr copolymerisierbaren Doppelbindungen, wie beispielsweise Diallylphthallat polymerisiert werden.

Als Metallsalze werden in der Regel 0,1 bis 30 Gew.-% bezogen auf die Copolymerisate an Metalloxiden, -Hydroxiden, -Halogeniden, - Carbonaten, -Hydrogensulfaten, -Sulfaten und/oder - Phosphaten zugesetzt.

Weiterhin enthalten die dilatanten Flüssigkeiten Verdünnungsmittel wie Alkohole, Glykole, Di- und Triglykole, Formamide und/oder Wasser. Für eine detailliertere Zusammensetzung des dilatanten Fluides wird auf die DE 30 25 562 A1 sowie die DE 39 17 456 A1 verwiesen.

Scherverdickende Fluide können auch einfache Dispersionen sein, welche ab einem bestimmten Feststoffanteil scherverdickende Eigenschaften aufweisen.

In einer weiter bevorzugten Ausführungsform kann das verwendete dilatante Fluid auch eine Dispersion aus Ethylenglykol (beispielsweise mit einer molaren Masse von 62 g/mol), Siliciumdioxid, bevorzugt Silica Gel mit einer Teilchengrößen von 5 bis -1000 nm und Oberflächen von 30 -bis 250 m²/g, mit einem Feststoffanteil von 10 - 80 Gew.-% und geeignete Stabilisatoren umfassen.

In einer bevorzugten Ausgestaltung ist der Auszugskörper linear bewegbar in der Aufnahme angeordnet. Dadurch wird eine gerichtete Funktionssicherung, Fixation, Korrektur und/oder Stützung eines Gelenks ermöglicht.

In einer bevorzugten Ausführungsform ist das mit dem dilatanten Fluid gefüllte Volumen durch einen Spalt zwischen der inneren Oberfläche der Aufnahme und einer äußeren Oberfläche des Auszugskörpers definiert. Dadurch ist der Teil des Auszugskörpers, welcher sich im Inneren der Aufnahme befindet, vollständig von dem dilatanten Fluid umgeben und kann mit diesem in Abhängigkeit von der Größe der auf den Auszugskörper wirkenden Kräfte Wechselwirkungen eingehen. Bei einer definierten geringen Kräfteeinwirkung kann der Auszugskörper in dem dilatanten Fluid nahezu uneingeschränkt bewegt werden. Bei hoher Krafteinwirkung und einem daraus resultierenden starken beziehungsweise abrupten Zug am Auszugskörper kommt es zu einem Überschreiten der kritischen Schergeschwindigkeit in dem dilatanten Fluid. Dies hat je nach verwendetem Fluid einen Anstieg der Zugspannung um den Faktor 100 bis zum Faktor 1000 zur Folge. Der Anstieg der Zugspannung hat eine vernetzende Wirkung auf das dilatante Fluid, wodurch der eine hohe Krafteinwirkung erfahrende Auszugskörper in dem dilatanten Fluid gehalten wird und so keine oder nur eine verminderte Bewegung erfährt.

In einer weiter bevorzugten Ausführungsform weist der Spalt ein Spaltmaß von 0,1 bis 100 mm, bevorzugt 0,1 bis 50 mm, besonders bevorzugt 0,1 bis 15 mm und ganz besonders bevorzugt 0,1 bis 5 mm auf. Abhängig von der Größe des Spaltmaßes kann die Rückhaltekraft anwendungsspezifisch eingestellt werden. Die gewünschte Scherverfestigung kann also je nach Anwendungsfall neben dem Feststoffanteil in dem Fluid, welcher durch Additive wie Stabilisatoren, Dispersionsmittel und/oder der gleichen bereitgestellt werden kann, auch über das Spaltmaß modifiziert beziehungsweise eingestellt werden.

Die Dimension des Spaltmaßes wirkt sich auch auf die Bauhöhe der orthopädischen Vorrichtung aus. So kann durch die Wahl eines geringen Spaltmaßes in Verbindung mit einer flachen Aufnahme sowie mit einem damit einhergehenden flachen Auszugskörper eine insgesamt flache orthopädische Vorrichtung erreicht werden. Solche besonders flachen orthopädischen Vorrichtungen erweisen sich als äußerst nutzerfreundlich, da sie aufgrund ihres geringen Platzbedarfes den Nutzer beim Tragen wenig stören. So lassen sich beispielsweise Kleidungsstücke leichter über solche flachen orthopädischen Vorrichtungen an- beziehungsweise ausziehen. Darüber hinaus kann eine flache Vorrichtung leichter in Kleidungsstücke wie beispielsweise Schuhwerk integriert werden.

In einer Weiterbildung weist der Spalt ein erstes Spaltmaß zwischen der inneren Oberfläche der Aufnahme und einer ersten und zweiten Oberfläche des Auszugskörpers, und ein zweites Spaltmaß zwischen der inneren Oberfläche der Aufnahme und Seitenflächen des Auszugskörpers auf. Dabei sind das erste und das zweite Spaltmaß unterschiedlich. Dadurch kann beispielsweise zwischen der inneren Oberfläche der Aufnahme und den Seitenflächen des Auszugskörpers ein geringes Spaltmaß gewählt werden, so dass die Seitenflächen des Auszugskörpers zur seitlichen Führung des Auszugskörpers in der Aufnahme dienen.

Die Wechselwirkungen des dilatanten Fluides mit dem Auszugskörper finden überwiegend zwischen dem dilatanten Fluid und der ersten und zweiten Oberfläche des Auszugskörpers statt. Entsprechend kann durch Veränderung des Spaltmaßes zwischen der inneren Oberfläche der Aufnahme und der ersten und zweiten Oberfläche des Auszugskörpers die Wechselwirkung zwischen dem dilatanten Fluid und dem Auszugskörper definiert werden.

In einer weiteren bevorzugten Weiterbildung umfasst die orthopädische Vorrichtung ein Mittel zum Zurückstellen des Auszugskörpers in eine Ausgangsposition in der Aufnahme. Dadurch kann die orthopädische Vorrichtung das betreffende Gelenk wieder in eine Ruheposition zurückführen, sofern keine Kräfte auf das Gelenk wirken. Folglich sollte die Ausgangsposition des Auszugskörpers in der Aufnahme derart gewählt werden, dass, wenn sich die orthopädische Vorrichtung in der Ausgangsposition befindet, das betroffene Gelenk in der bevorzugten Ruheposition ist.

Des Weiteren kann durch das Mittel zum Zurückstellen auch die von dem Nutzer gewollte Einnahme der Ruheposition unterstützt werden.

Ist der Auszugskörper durch das Mittel zum Zurückstellen wieder in die Ausgangsposition überführt worden, so steht der orthopädischen Vorrichtung und insbesondere dem Auszugskörper die gesamte Hublänge zur adaptiven Bewegungsbegrenzung des entsprechenden Gelenks zur Verfügung.

In einer weiter bevorzugten Ausgestaltung umfasst das Mittel zum Zurückstellen einen zwischen der Aufnahme und dem Auszugskörper angeordneten elastischen Kunststoff, eine Feder, zwei Permanentmagneten und/oder eine Dichtung. Durch die Verwendung eines elastischen Kunststoffes kann die Spannkraft dieses elastischen Kunststoffes dazu verwendet werden, den Auszugskörper in die Ausgangsposition zurückzustellen. Ein elastischer Kunststoff, beispielsweise in Form eines Gummibandes, stellt ein einfaches kostengünstiges Mittel zum Zurückstellen dar. Bei der Verwendung einer Feder als Mittel zum Zurückstellen des Auszugskörpers in die Ausgangsposition wird die Rückstellung durch die Federkraft der Feder bestimmt. Die Verwendung von zwei Permanentmagneten als Mittel zum Zurückstellen des Auszugskörpers, wobei ein Permanentmagnet an dem Auszugskörper und ein weiterer an der Aufnahme angeordnet ist, hat den Vorteil, dass die Zurückstellung des Auszugskörpers in die Ausgangsposition in der Aufnahme kontaktlos erreicht werden kann. Eine dichte Verbindung zwischen dem Auszugskörper und der Aufnahme ist somit nicht erforderlich.

Durch die Verwendung der Dichtung als Mittel zum Zurückstellen des Auszugskörpers können gegenüber anderen Lösungen Bauteile beziehungsweise Herstellkosten eingespart werden. Darüber hinaus weist eine Dichtung, welche in der Regel umlaufend an ihren Enden mit der Aufnahme sowie dem Auszugskörper verbunden ist, eine gleichmäßig verteilte Übertragung der Rückstellkraft auf den Auszugskörper auf.

In einer Weiterbildung umfassen die Oberflächen des Auszugkörpers (3) und/oder der Aufnahme (2), welche mit dem dilatanten Fluid (4) in Verbindung stehen und senkrecht zur Normalen der Auszugsrichtung liegen eine Strukturierung. Dadurch werden die mit dem dilatanten Fluid in Verbindung stehenden Oberflächen des Auszugskörpers beziehungsweise der Aufnahme vergrößert. Größere Oberflächen des Auszugskörpers beziehungsweise der Aufnahme haben eine größere Wechselwirkung dieser Oberflächen mit dem dilatanten Fluid zur Folge. Folglich können durch die Strukturierung der Oberflächen im Vergleich zu unstrukturierten Oberflächen mit gleichen Abmessungen, höhere Scherkräfte und somit ein früher einsetzender Dilantanzsprung, bei welchem eine deutliche Zunahme der Viskosität zu verzeichnen ist, erreicht werden.

Für die Viskosität des dilatanten Fluides bedeutet das, dass durch die Strukturierung der Oberflächen die gleiche Viskosität bei einem geringen Spaltmaß möglich ist. Dadurch kann wiederum Bauraum eingespart werden, wodurch die orthopädische Vorrichtung insgesamt nutzerfreundlicher wird.

In einer weiter bevorzugten Ausführungsform umfasst die Strukturierung der Oberflächen Ausprägungen, Rippen, Rillen und/oder Noppen oder besteht aus einer rauen Oberfläche. Dadurch kann der oben beschriebene Effekt der Strukturierung erzielt werden. Entsprechend könne durch Ausprägungen, Rippen, Rillen und/oder Noppen auf dem Auszugskörper und/oder der Aufnahme die mit dem dilatanten Fluid in Verbindung stehenden Oberflächen vergrößert werden. Größere Oberflächen des Auszugskörpers beziehungsweise der Aufnahme können größere Wechselwirkungen dieser Oberflächen mit dem dilatanten Fluid eingehen, wodurch höhere Scherkräfte und somit ein früher einsetzender Dilantanzsprung eintreten können.

In einer Weiterbildung umfasst die Aufnahme eine Öffnung, aus welcher der Auszugskörper herausragt. Dadurch kann an dem herausragenden Teil des Auszugskörpers eine Haltevorrichtung beziehungsweise der Teil einer Haltevorrichtung angebracht werden, welcher eine körperteilumschließende, kraftaufnehmende, kraftübertragende und fixierende Funktion aufweist.

Dabei ist die Haltevorrichtung beziehungsweise der Teil der Haltevorrichtung an einem Körperbereich angeordnet, welcher an das in seiner Bewegung adaptiv zu begrenzende Gelenk angrenzt.

In einer weiter bevorzugten Ausführungsform weist die Aufnahme eine Dichtung zur Abdichtung der Bewegung des Auszugskörpers auf. Dadurch kann sichergestellt werden, dass das dilatante Fluid beim Ein- und Ausfahren des Auszugskörpers stets im Inneren der Aufnahme gehalten wird. Entsprechend sorgt die Dichtung dafür, dass auf der Oberfläche des Teils des Auszugskörpers, welcher sich außerhalb der Aufnahme befindet, keine Rückstände des dilatanten Fluides verbleiben. Somit kann sichergestellt werden, dass das dilatante Fluid die unmittelbare Umgebung der orthopädischen Vorrichtung nicht kontaminiert und insbesondere der Nutzer nicht mit dem dilatanten Fluid in Kontakt gerät. Dabei kann die Dichtung beispielsweise in Form einer umlaufenden Dichtlippe ausgeführt sein.

Darüber hinaus verhindert die Dichtung, dass die sich in der Aufnahme befindliche Menge an dilatantem Fluid durch die Bewegungen des Auszugskörpers reduziert wird. So wird das dilatante Fluid durch den Einsatz der orthopädischen Vorrichtung nicht verbraucht und muss nicht beziehungsweise seltener nachgefüllt werden.

In einer weiter bevorzugten Weiterbildung ist die Dichtung an einem ersten Ende mit der Aufnahme verbunden und an einem zweiten Ende mit dem Auszugskörper verbunden, wobei die Dichtung balgartig bewegbar ist. Dadurch ist die Dichtung dazu in der Lage, den Bewegungen des Auszugskörpers zu folgen. Entsprechend entfaltet sich der Balg zwischen dem ersten und dem zweiten Ende der Dichtung, wenn der Auszugskörper aus der Aufnahme heraus bewegt wird. Wird der Auszugskörper in die Aufnahme hinein bewegt, so kommt es hingegen zu einer balgartigen Faltung der Dichtung.

Weder das zweite noch das erste Ende der Dichtung muss dabei gegen einen beweglichen Körper abdichten. Das heißt, es ist keine umlaufende Dichtlippe erforderlich, zu welcher sich ein Körper relativ bewegt. Vielmehr besteht zwischen den beiden Enden der Dichtung sowie der Aufnahme beziehungsweise dem Auszugskörper eine kraft- oder eine reibschlüssige Verbindung.

In einer weiteren, nicht vom unabhängigen Anspruch erfassten Ausführungsform, sind die Aufnahme und/oder der Auszugskörper aus Kunststoff, bevorzugt aus thermoplastischem Kunststoff, besonders bevorzugt aus Polypropylen,
Polyethylen und/oder Polyurethan gefertigt. Die Verwendung von Kunststoff bietet die für den Einsatzbereich der orthopädischen Vorrichtung nötigen Festigkeiten und Steifigkeiten bei gleichzeitig geringem Gewicht. Ein geringes Gewicht der orthopädischen Vorrichtung kann sich insbesondere vorteilhaft auf den Tragekomfort auswirken. Darüber hinaus verfügt Kunststoff in der Regel über eine hohe Biokompatibilität, welche gerade bei ständigem oder länger andauerndem Hautkontakt von Vorteil sein kann. Ferner werden die eingesetzten Kunststoffe auch den medizinischen Hygiene Anforderungen gerecht. Des Weiteren ermöglicht die Verwendung von Kunststoff auch die Herstellung mittels Zweikomponenten-Spritzgussverfahren. Dadurch kann beispielsweise eine Dichtung bereits bei der Herstellung in den Auszugskörper integriert werden.

In einer bevorzugten Weiterbildung umfasst der Auszugkörper Verstärkungsfasern, bevorzugt Naturfasern, Aramidfasern, Glasfasern, Kohlefasern oder andere Kunststofffasern. Dadurch kann die Bewegungsfreiheit der orthopädischen Vorrichtung erhöht werden. So können die Teile der Fasern des Auszugskörpers, welche aus der Aufnahme heraus ragen sich in nahezu beliebiger Richtung erstrecken. So ist eine orthopädische Vorrichtung mit einem faserförmigen Auszugskörper insbesondere für Körperteile geeignet, welche einer hohen Bewegungsfreiheit bedürfen.

Darüber hinaus kann durch einen faserförmigen Auszugskörper sowohl Bauraum als auch Gewicht eingespart werden. Da die Fasern lediglich auf Zug belastet werden können, bedarf es in der Regel einem Mittel zum Zurückstellen des Auszugskörpers.

In einer Weiterbildung umfassen die Aufnahme und/oder der Auszugkörper eine Haltevorrichtung, welche an einem Körperbereich anbringbar ist. Dadurch kann ein Kraftfluss über die orthopädische Vorrichtung von einem Körperbereich auf einen anderen Körperbereich geleitet werden, wobei ein sich zwischen den beiden Körperbereichen befindliches Gelenk entlastet werden kann. Auf Grund der genannten Eigenschaften der orthopädischen Vorrichtung kann die relative Bewegung der Körperbereiche zueinander und somit die Bewegung des zwischen den Körperbereichen befindlichen Gelenks adaptiv begrenzt werden.

Des Weiteren kann durch das Anbringen der Haltevorrichtungen eine Ausgangsposition der orthopädischen Vorrichtung am Körper des Anwenders festgelegt werden.

### Kurze Beschreibung der Figuren

Bevorzugte weitere Ausführungsformen und Aspekte der vorliegenden Erfindung werden durch die nachfolgende Beschreibung der Figuren näher erläutert. Dabei zeigen:
- Figur 1: schematisch eine Draufsicht eines Schnitts durch die orthopädischen Vorrichtung,
- Figur 2: schematisch eine Querschnittansicht der orthopädischen Vorrichtung aus Figur 1,
- Figur 3: schematisch die orthopädischen Vorrichtung aus Figur 1 in einer Anordnung zur adaptiven Bewegungsbegrenzung eines Sprunggelenks,
- Figur 4: schematisch eine orthopädischen Vorrichtung mit zwei Aufnahmen, und
- Figur 5: schematisch die orthopädischen Vorrichtung aus Figur 3 in einer Anordnung zur adaptiven Bewegungsbegrenzung eines Sprunggelenks.

### Detaillierte Beschreibung bevorzugter Ausführungsbeispiele

Im Folgenden werden bevorzugte Ausführungsbeispiele anhand der Figuren beschrieben. Dabei werden gleiche, ähnliche oder gleichwirkende Elemente mit identischen Bezugszeichen bezeichnet. Um Redundanzen zu vermeiden, wird auf eine wiederholte Beschreibung dieser Elemente in der nachfolgenden Beschreibung teilweise verzichtet.

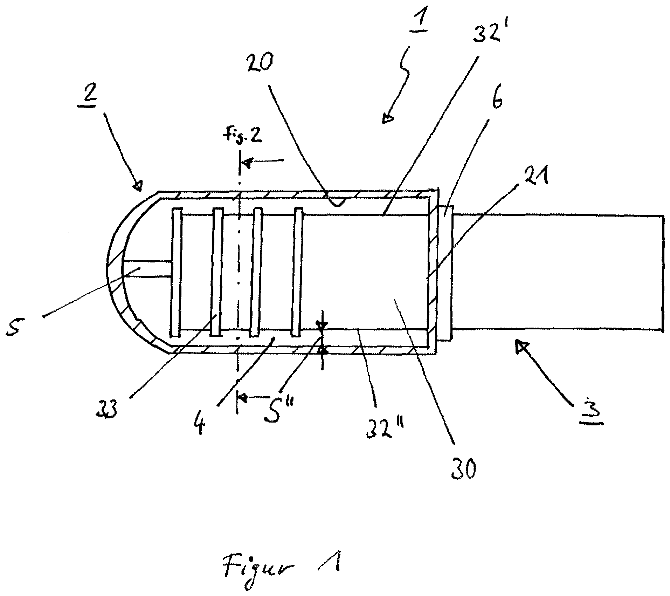

Figur 1 zeigt schematisch eine orthopädische Vorrichtung 1 zur Begrenzung der Bewegung eines zwischen einem ersten und einem zweiten Körperbereich angeordneten Gelenks. Dabei umfasst die Vorrichtung 1 eine Aufnahme 2, welche an dem ersten Körperbereich fixiert werden kann. Darüber hinaus zeigt die Figur 1 einen Auszugskörper 3, welcher an dem zweiten Körperbereich fixiert werden kann und sich zum Teil in das Innere der Aufnahme 2 erstreckt. Das zwischen dem Auszugskörper 3 und der inneren Oberfläche 20 der Aufnahme 2 ergebende Volumen ist mit einem dilatanten Fluid 4 gefüllt. Durch Krafteinwirkung auf die orthopädische Vorrichtung kann es so zu einer Wechselwirkung zwischen dem dilatanten Fluid 4 und der Oberfläche des Auszugskörpers 3 kommen, wodurch die Bewegung des Auszugskörpers 3 relativ zur Aufnahme 2 beeinflusst werden kann.

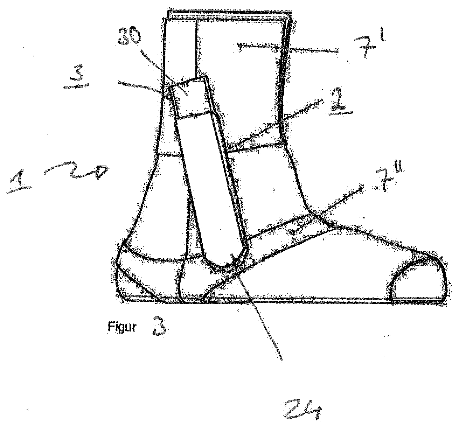

Die in Figur 1 gezeigte orthopädische Vorrichtung 1 zur adaptiven Bewegungsbegrenzung eines zwischen zwei Körperbereichen angeordneten Gelenks umfasst die Aufnahme 2, welche an einer beispielsweise in Figur 3 gezeigten Haltevorrichtung 7',7" gelagert ist. Über die Haltevorrichtung 7',7" kann die Aufnahme 2 an einem Körperbereich eines Anwenders fixiert werden.

Die Aufnahme 2 ist derart dimensioniert, dass sie in ihrem Inneren einen Auszugskörper 3 aufnehmen kann. Zur Aufnahme dieses Auszugskörpers 3 befindet sich an einem Ende der Aufnahme 2 eine Öffnung 21.

Die Aufnahme 2 ist aus Kunststoff gefertigt, welcher insbesondere fluidundurchlässig ist. Bevorzugt kommt ein thermoplastischer Kunststoff zum Einsatz, wobei es sich bei diesem um Polypropylen, Polyethylen und/oder Polyurethan halten kann.

Figur 1 zeigt weiterhin den Auszugskörper 3, welcher sich teilweise im Inneren der Aufnahme 2 befindet. Am Ende des Teils des Auszugskörpers 3, welcher aus der Aufnahme 2 herausragt, kann der Auszugskörper 3 über eine Haltevorrichtung 7',7", wie zum Beispiel in Figur 3 gezeigt ist, mit einem Körperbereich eines Anwenders verbunden werden.

Der Auszugskörper 3 ist in der Aufnahme 2 zum Beispiel in einer Schienenführung (nicht gezeigt) linear bewegbar gehalten. Dabei kann der Auszugskörper 3 durch die Öffnung 21 der Aufnahme 2 in diese ein- beziehungsweise aus dieser ausgefahren werden.

Der Auszugskörper 3 besitzt eine quaderförmige Form und weist eine erste Oberfläche 30, eine zweite Oberfläche 31 (in Figur 1 nicht gezeigt) und Seitenflächen 32', 32" auf. Zwischen der inneren Oberfläche 20 der Aufnahme 2 und der ersten Oberfläche 30, der zweiten Oberfläche 31 sowie den beiden Seitenflächen 32', 32" des Auszugskörpers 3 besteht ein Spalt mit einem Spaltmaß S. Dieses Spaltmaß S ergibt sich aus der Volumendifferenz des Innenvolumens der Aufnahme und des verdrängenden Volumens des Teils des Auszugskörpers, welcher sich im Inneren der Aufnahme befindet.

Darüber hinaus ist das Volumen zwischen der Aufnahme 2 und dem Auszugskörper 3 mit einem dilatanten Fluid 4 gefüllt. Wird der Auszugskörper 3 in die Aufnahme 2 hinein oder aus der Aufnahme 2 heraus bewegt, so wird die Bewegung des Auszugskörpers 3 von den Eigenschaften des dilatanten Fluides 4 beeinflusst. Wird die orthopädische Vorrichtung 1 folglich unter geringer Krafteinwirkung ineinander beziehungsweise auseinander geschoben, so lässt es das dilatante Fluid 4 zu, dass sich der Auszugskörper 3 in dem dilatanten Fluid 4 bewegt. Je größer die auf die orthopädische Vorrichtung 1 wirkenden Kräfte sind, welche die Aufnahme 2 und den Auszugskörper 3 ineinander beziehungsweise auseinander bewegen, umso größer sind die zwischen dem Auszugskörper 3 und dem dilatanten Fluid 4 auftretenden Scherkräfte. Mit dem Anstieg der Scherkräfte steigt auch die Viskosität des dilatanten Fluides. Dabei weist das dilatante Fluid beispielsweise einen Dilatanzsprung auf, bei welchem eine deutliche Zunahme der Viskosität zu verzeichnen ist. So lassen sich die Aufnahme 2 und der Auszugskörper 3 der orthopädischen Vorrichtung 1 bei einer in dem dilatanten Fluid 4 herrschenden hohen Viskosität, insbesondere jenseits des Dilantanzsprungs, nicht mehr oder nur noch geringfügig zueinander bewegen.

Figur 1 zeigt ferner, dass der Auszugskörper 3 eine Strukturierung 33 aufweist. Die Strukturierung 33 verhilft dem Auszugskörper 3 zu einer größeren Oberfläche. In Figur 1 ist die Strukturierung 33 in Form von Rillen ausgebildet. Dabei handelt es sich bei den strukturierten Oberflächen um Oberflächen, welche senkrecht zur Normalen der Auszugsrichtung angeordnet sind. Alternativ sind auch Rippen, Noppen oder dergleichen denkbar. Durch die Vergrößerung der Oberfläche des Teils des Auszugskörpers 3, welcher sich in Kontakt mit dem dilatanten Fluid 4 befindet, können die Eigenschaften der orthopädischen Vorrichtung dahingehend verändert werden, dass bei vergleichsweise geringen Scherkräften bereits ein hoher Anstieg der Viskosität des dilatanten Fluides 4 vorliegt. Dadurch kann beispielsweise ein Dilatanzsprung dahingehend verschoben werden, dass er früher, d.h. bei geringeren Scherkräften auftritt, als bei einem Auszugskörper ohne Strukturierung.

Figur 1 zeigt weiterhin ein Mittel zum Zurückstellen 5 des Auszugskörpers 3 in eine Ausgangsposition in der Aufnahme 2. Abhängig von dem zu unterstützenden Gelenk kann das Mittel zum Zurückstellen 5 nötig sein, um das Gelenk, nachdem es eine Auslenkung erfahren hat, wieder in eine Ruheposition zurückzuführen. Dabei ist das Mittel zum Zurückstellen 5 derart gewählt, dass das Gelenk eine schonende Zurückstellung erfährt beziehungsweise dass der Auszugskörper 3 so langsam durch das dilatante Fluid 4 geführt wird, dass kein erheblicher Anstieg der Viskosität in dem dilatanten Fluid 4 hervorgerufen wird.

Das in Figur 1 gezeigte Mittel zum Zurückstellen 5 ist ein elastischen Kunststoff in Form eines Gummibandes mit den oben beschriebenen Eigenschaften. Alternativ kann das Mittel zum Zurückstellen auch durch eine Feder, durch ein Paar Permanentmagneten, wobei der eine Permanentmagnet am Ende der Aufnahme 2 und der andere Permanentmagnet am gegenüberliegenden Ende des Auszugskörpers 3 angeordnet ist, oder durch eine elastische Dichtung ausgebildet sein. Darüber hinaus kann die Rückstellung des Auszugskörpers 3 auch über einen in der Aufnahme 2 herrschenden Unterdruck erfolgen. Der Unterdruck entsteht dabei beim Herausziehen des Auszugskörpers 3 bei einer entsprechenden Abdichtung.

Darüber hinaus ist der Figur 1 eine Dichtung 6 zu entnehmen, welche auf der Öffnung 21 der Aufnahme 2 sitzt und den Innenraum der Aufnahme 2 gegen die äußere Umgebung abdichtet. Die Dichtung 6 umläuft dabei die Öffnung 21 der Aufnahme 2 beziehungsweise die erste Oberfläche 30, die zweite Oberfläche 31 sowie die Seitenflächen 32', 32" des Auszugskörpers 3. Dabei kann der Auszugskörper 3 relativ zur Dichtung 6 bewegt werden. Eine umlaufende Dichtlippe der Dichtung 6 verhindert dabei, dass dilatantes Fluid 4 aus dem Inneren der Aufnahme 2 durch die Öffnung 21 austritt.

Alternativ kann die Dichtung 6 auch balgförmig ausgebildet sein. Dabei ist ein erstes Ende des Dichtungsbalges fest mit der Aufnahme 2 und ein zweites Ende des Dichtungsbalges fest mit dem Auszugskörper 3 verbunden. Wird der Auszugskörper 3 aus der Aufnahme 2 heraus bewegt, so vergrößert sich der Abstand zwischen dem ersten und dem zweiten Ende des Dichtungsbalges. Entsprechend folgt der Dichtungsbalg den Bewegungen der orthopädischen Vorrichtung 1 beziehungsweise des Auszugskörpers 3.

Darüber hinaus kann eine Balgdichtung zusätzlich die Funktion des Mittels zum Zurückstellen 5 übernehmen. In diesem Fall weist der Balgkörper der Dichtung eine Elastizität auf, die es ermöglicht, nach Auslenkung der orthopädischen Vorrichtung 1 wieder eine Ruheposition einzunehmen, indem sich das erste Ende und das zweite Ende der Balgdichtung wieder einander annähern. Als Dichtmaterial kommen dabei beispielsweise elastische Gummis oder Polymere zum Einsatz.

Auch der Auszugskörper 3 ist aus Kunststoff gebildet. Dabei kommen bevorzugt thermoplastische Kunststoffe, wie zum Beispiel Polypropylen, Polyethylen oder Polyurethan zum Einsatz.

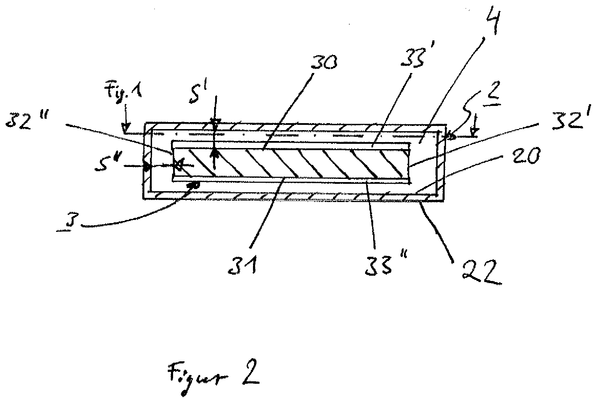

Figur 2 zeigt eine um 90° gedrehte Schnittansicht der orthopädischen Vorrichtung 1 aus Figur 1. Dabei zeigt Figur 2 die Anordnung des Auszugskörpers 3 im Inneren der Aufnahme 2.

Die erste Oberfläche 30 und die zweite Oberfläche 31 des Auszugskörpers 3 sind jeweils um das Spaltmaß S' von der inneren Oberfläche 20 der Aufnahme 2 beabstandet. Die Seitenflächen 32', 32" des Auszugskörpers 3 sind um das Spaltmaß S" von der inneren Oberfläche 20 der Aufnahme 2 beabstandet. Dabei sind die Spaltmaße S' und S" unterschiedlich.

Figur 3 zeigt die orthopädische Vorrichtung aus Figur 1 in einer Anordnung zur adaptiven Bewegungsbegrenzung eines Sprunggelenks. Dabei ist die orthopädische Vorrichtung 1 über die Haltevorrichtungen 7', 7" an Körperbereiche fixiert, welche an das Sprunggelenk angrenzen.

Das Ende des Teils des Auszugskörpers 3, welches aus der Aufnahme 2 herausragt, ist an der Haltevorrichtung 7' befestigt.

Die äußere Oberfläche 22 der Aufnahme 2 ist im Bereich eines geschlossenen Endes 24 an der Haltevorrichtung 7" befestigt.

Die Haltevorrichtung 7' und 7" wird derart am Körper des Anwenders angebracht, dass sich das betroffene Gelenk dazwischen befindet.

Der Auszugskörper 3 beziehungsweise die Aufnahme 2 sind jeweils auf die Haltevorrichtung 7' beziehungsweise 7" aufgeklebt. Alternativ können der Auszugskörper 3 beziehungsweise die Aufnahme 2 auch mit den Haltevorrichtungen 7', 7" vernäht, verschraubt, verschnürt oder geklettet werden. Darüber hinaus ist auch die Verbindung über ein Kugelgelenk möglich.

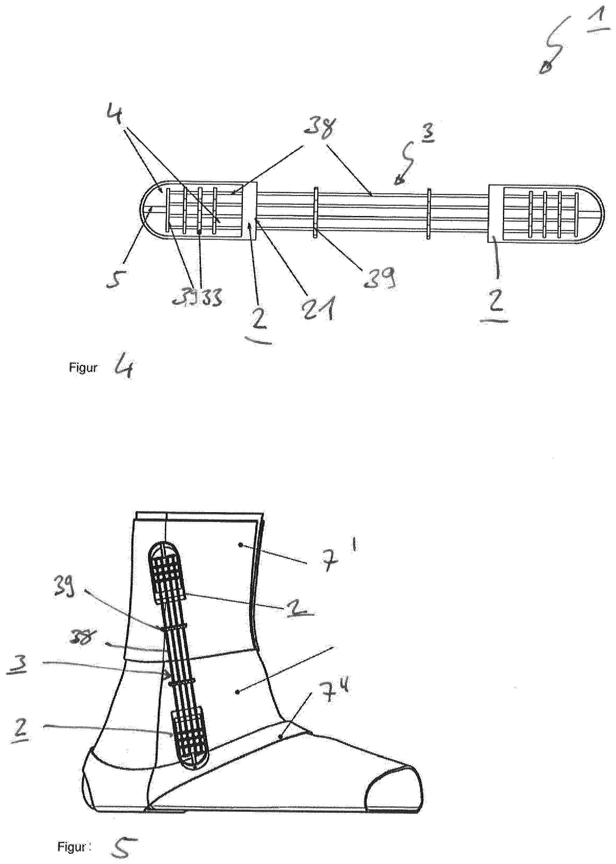

Figur 4 zeigt schematisch eine orthopädische Vorrichtung 1, bei welcher beide Enden des Auszugskörpers 3 in jeweils einer Aufnahme 2 gehalten sind. Der Aufbau der Aufnahme 2 gleicht der Aufnahme aus Figur 1.

Ferner besteht der Auszugskörper 3 aus einzelnen Auszugsstäben 38, welche über Querstreben 39 zusammengehalten werden. Im Inneren der Aufnahmen 2 kann dadurch das dilatante Fluid 4 auch in die Zwischenräume der Auszugsstäbe 38 fließen. Dabei sind die Auszugsstäbe aus thermoplastischem Kunststoff, besonders bevorzugt aus Polypropylen, Polyethylen und/oder Polyurethan, gefertigt (nicht vom unabhängigen Anspruch erfasst).

Alternativ können anstatt der Auszugsstäbe 38 auch Faserstränge aus Verstärkungsfasern eingesetzt werden. Auch die Faserstränge werden durch Querstreben 39 zusammengehalten. Da die Fasern nur auf Zug belastet werden können, spielen bei einer derartigen Ausführungsform die Mittel zum Zurückstellen 5 des Auszugskörpers 3 eine gesonderte Rolle, da der Auszugskörper 3 beziehungsweise die Faserstränge ohne die Mittel zum Zurückstellen 5 nicht dazu in der Lage sind, nach einer Auslenkung durch auf die orthopädische Vorrichtung 1 einwirkende Kräfte wieder eine Ausgangsposition einzunehmen.

Als Fasern können Naturfasern, Aramidfasern, Glasfasern, Kohlefasern oder andere Kunststofffasern zum Einsatz kommen.

Die in Figur 4 gezeigten Aufnahmen 2 weisen mehrere Öffnungen 21 auf, durch welche jeweils ein Auszugsstab geführt ist. Dabei ist in jeder Öffnung 21 eine Dichtung 6 angeordnet, welche verhindert, dass dilatantes Fluid 4 aus dem Inneren der Aufnahmen 2 bei Bewegung der Auszugsstäbe 38 austritt.

Figur 5 zeigt die orthopädische Vorrichtung 1 aus Figur 4 in einer Anordnung zur Stabilisierung eines Sprunggelenks. Dabei sind die Aufnahmen 2, wie in Figur 3 gezeigt, an den Haltevorrichtungen 7' und 7" befestigt.

Soweit anwendbar, können alle einzelnen Merkmale, die in den einzelnen Ausführungsbeispielen dargestellt sind, miteinander kombiniert und/oder ausgetauscht werden, ohne den Bereich der Erfindung zu verlassen.

### Bezugszeichenliste

- 1: Vorrichtung

- 2: Aufnahme
- 20: Innere Oberfläche
- 21: Öffnung

- 3: Auszugskörper
- 30: Erste Oberfläche
- 31: Zweite Oberfläche
- 32',32": Seitenfläche
- 33: Strukturierung

- 4: Dilatantes Fluid

- 5: Mittel zum Zurückstellen

- 6: Dichtung

- 7',7": Haltevorrichtung

- S: Spaltmaß
- S': Spaltmaß
- S": Spaltmaß

## Patentansprüche

1. Orthopädische Vorrichtung (1) zur Begrenzung der Bewegung eines zwischen einem ersten und einem zweiten Körperbereich angeordneten Gelenks, umfassend zumindest eine an dem ersten Körperbereich fixierbare Aufnahme (2) und einen an dem zweiten Körperbereich fixierbaren und relativ zu der Aufnahme (2) bewegbaren Auszugskörper (3), wobei zwischen der Aufnahme (2) und dem Auszugskörper (3) ein mit einem dilatanten Fluid (4) gefülltes Volumen vorgesehen ist,
**dadurch gekennzeichnet, dass**
der Auszugskörper (3) faserförmig Ist und Verstärkungsfasern, bevorzugt Naturfasern, Aramidfasern, Glasfasern, Kohlefasern oder andere Kunststofffasern, umfasst.

2. Orthopädische Vorrichtung (1) gemäß Anspruch 1, **dadurch gekennzeichnet, dass** der Auszugskörper (3) linear bewegbar in der Aufnahme (2) angeordnet ist.

3. Orthopädische Vorrichtung (1) gemäß Anspruch 1 oder 2, **dadurch gekennzeichnet, dass** das mit dem dilatanten Fluid (4) gefüllte Volumen durch einen Spalt zwischen der inneren Oberfläche (20) der Aufnahme (2) und einer äußeren Oberfläche (30) des Auszugskörpers (3) definiert ist.

4. Orthopädische Vorrichtung (1) gemäß Anspruch 3, **dadurch gekennzeichnet, dass** der Spalt ein Spaltmaß (S',S") von 0,1 bis 100 mm, bevorzugt 0,1 bis 50 mm, besonders bevorzugt 0,1 bis 15 mm, und ganz besonders bevorzugt 0,1 bis 5 mm aufweist.

5. Orthopädische Vorrichtung (1) gemäß Anspruch 3 oder 4, **dadurch gekennzeichnet, dass** der Spalt ein erstes Spaltmaß (S') zwischen der inneren Oberfläche der Aufnahme (2) und einer ersten und zweiten Oberfläche (30, 31) des Auszugkörpers (3), und ein zweites Spaltmaß (S") zwischen der inneren Oberfläche der Aufnahme (2) und Seitenflächen (32',32") des Auszugkörpers (3) aufweist, und erstes und zweites Spaltmaß unterschiedlich sind.

6. Orthopädische Vorrichtung (1) gemäß einem der vorhergehenden Ansprüche, **dadurch gekennzeichnet, dass** die Vorrichtung ein Mittel zum Zurückstellen (5) des Auszugskörpers (3) in eine Ausgangsposition in der Aufnahme (2) umfasst.

7. Orthopädische Vorrichtung (1) gemäß Anspruch 6, **dadurch gekennzeichnet, dass** das Mittel zum Zurückstellen (5) ein zwischen der Aufnahme (2) und dem Auszugskörper (3) angeordneten elastischen Kunststoff, eine Feder, zwei Permanentmagneten und/oder eine Dichtung umfasst.

8. Orthopädische Vorrichtung (1) gemäß einem der vorhergehenden Ansprüche, **dadurch gekennzeichnet, dass** die Oberflächen (30, 31, 32', 33") des Auszugkörpers (3) und/oder der Aufnahme (2), welche mit dem dilatanten Fluid (4) in Verbindung stehen und senkrecht zur Normalen der Auszugsrichtung liegen, eine Strukturierung (33) umfassend

9. Orthopädische Vorrichtung (1) gemäß Anspruch 8, **dadurch gekennzeichnet, dass** die Strukturierung (33) der Oberfläche Ausprägungen, Rippen, Rillen, Noppen und/oder eine raue Oberfläche umfasst.

10. Orthopädische Vorrichtung (1) gemäß einem der vorhergehenden Ansprüche, **dadurch gekennzeichnet, dass** die Aufnahme (2) eine Öffnung (21) umfasst, aus welcher der Auszugskörper (3) heraus ragt.

11. Orthopädische Vorrichtung (1) gemäß einem der vorhergehenden Ansprüche, **dadurch gekennzeichnet, dass** die Aufnahme (2) eine Dichtung (6) zur Abdichtung der Bewegung des Auszugskörpers (3) aufweist.

12. Orthopädische Vorrichtung (1) gemäß Anspruch 11, **dadurch gekennzeichnet, dass** die Dichtung (6) an einem ersten Ende mit der Aufnahme (2) verbunden ist und an einem zweiten Ende mit dem Auszugskörper (3) verbunden ist, wobei die Dichtung (6) balgartig bewegbar ist.

13. Orthopädische Vorrichtung (1) gemäß einem der vorhergehenden Ansprüche, **dadurch gekennzeichnet, dass** die Aufnahme und/oder der Auszugkörper aus Kunststoff, bevorzugt aus thermoplastischem Kunststoff, besonders bevorzugt aus Polypropylen, Polyethylen und/oder Polyurethan gefertigt sind.

14. Orthopädische Vorrichtung gemäß einem der vorhergehenden Ansprüche, **dadurch gekennzeichnet, dass** die Aufnahme (2) und/oder der Auszugkörper (3) eine Haltevorrichtung (7) umfassen, welche an einem Körperbereich anbringbar Ist.

15. Orthopädische Vorrichtung gemäß einem der vorhergehenden Ansprüche, **dadurch gekennzeichnet, dass** das dilatante Fluid eine Dispersion aus Ethylenglykol, Siliciumdioxid, bevorzugt Silica Gel mit einer Teilchengrößen von 5 bis -1000 nm und Oberflächen von 30 -bis 250 m²/g, einen Feststoffenteil von 10 - 80 Gew.-%, und Stabilisatoren umfasst.

## Claims

1. Orthopedic device (1) for the limitation of the movement of a joint disposed between a first and a second body portion, comprising at least a reception (2) fixable to the first body portion and a pull-out body (3) fixable to the second body portion and movable relative to the reception (2), wherein a volume filled with shear thickening fluid (4) is provided between the reception (2) and the pull-out body (3),
**characterized in that**
the pull-out body (3) is fibrous and comprises reinforcement fibers, preferably natural fibers, aramid fibers, glass fibers, carbon fibers or other synthetic fibers.

2. Orthopedic device (1) according to claim 1, **characterized in that** the pull-out body (3) is linearly movable disposed in the reception (2).

3. Orthopedic device (1) according to claim 1 or 2, **characterized in that** the volume filled with shear thickening fluid (4) is defined by a gap between the inner surface (20) of the reception (2) and an outer surface (30) of the pull-out body (3).

4. Orthopedic device (1) according to claim 3, **characterized in that** the gap has a clearance (S', S") of 0.1 to 100 mm, preferably 0.1 to 50 mm, more preferably 0.1 to 15 mm and even more preferably 0.1 to 5 mm.

5. Orthopedic device (1) according to claims 3 or 4, **characterized in that** the gap has a first clearance (S') between the inner surface of the reception (2) and the first and second surfaces (30, 31) of the pull-out body (3), and a second clearance (S") between the inner surface of the reception (2) and lateral surfaces (32', 32") of the pull-out body (3), and the first and second clearance differ from each other.

6. Orthopedic device (1) according to one of the preceding claims, **characterized in that** the device comprises a means (5) to reset the pull-out body (3) into a starting position inside the reception (2).

7. Orthopedic device (1) according to claim 6, **characterized in that** the reset means (5) comprise an elastic synthetic material, a spring, two permanent magnets and/or a sealing disposed between the reception (2) and the pull-out body (3).

8. Orthopedic device (1) according to one of the preceding claims, **characterized in that** the surfaces (30, 31, 32', 33") of the pull-out body (3) and/or the reception (2), which are in communication with the shear thickening fluid (4) and are disposed vertically to the normal of the pull-out direction, comprise a structure (33).

9. Orthopedic device (1) according to claim 8, **characterized in that** the structure (33) of the surface comprises shapings, fins, grooves, nubs and/or a rough surface.

10. Orthopedic device (1) according to one of the preceding claims, **characterized in that** the reception (2) comprises an opening (21), out of which the pull-out body (3) protrudes.

11. Orthopedic device (1) according to one of the preceding claims, **characterized in that** the reception (2) has a sealing (6) for sealing the movement of the pull-out body (3).

12. Orthopedic device (1) according to claim 11, **characterized in that** the sealing (6) at a first end is connected to the reception (2) and at a second end is connected to the pull-out body (3), wherein the sealing (6) is movable like a bellows.

13. Orthopedic device (1) according to one of the preceding claims, **characterized in that** the reception and/or the pull-out body are made of synthetic material, preferably out of thermoplastic synthetic material, more preferably out of polypropylene, polyethylene and/or polyurethane.

14. Orthopedic device according to one of the preceding claims, **characterized in that** the reception (2) and/or the pull-out body (3) comprise a holding device (7), which is mountable to a body portion.

15. Orthopedic device according to one of the preceding claims, **characterized in that** the shear thickening fluid comprises a dispersion of ethylene glycol, silicon dioxide, preferably silica gel with a particle size of 5 to 1000 nm and surfaces of 30 to 250 m²/g, a solid content of 10 to 80 %-by weight and stabilizers.

## Revendications

1. Dispositif orthopédique (1) permettant de limiter le mouvement d'une articulation disposée entre une première et une deuxième partie du corps, comprenant au moins un logement (2) pouvant être fixé à la première partie du corps et un corps de rallonge (3) pouvant être fixé à la deuxième partie du corps et mobile par rapport au logement (2), un volume rempli de fluide (4) dilaté étant prévu entre le logement (2) et le corps de rallonge (3), **caractérisé en ce que** le corps de rallonge (3) est en forme de fibre et comprend des fibres de renforcement, de façon préférée des fibres naturelles, des fibres d'aramide, des fibres de verre, des fibres de carbone ou d'autres fibres de matière synthétique.

2. Dispositif orthopédique (1) selon la revendication 1, **caractérisé en ce que** le corps de rallonge (3) est disposé de façon à pouvoir être déplacé de façon linéaire dans le logement (2).

3. Dispositif orthopédique (1) selon la revendication 1 ou 2, **caractérisé en ce que** le volume rempli de fluide (4) dilaté est défini par un interstice existant entre la surface (20) intérieure du logement (2) et une surface (30) extérieure du corps de rallonge (3).

4. Dispositif orthopédique (1) selon la revendication 3, **caractérisé en ce que** l'interstice présente une dimension d'interstice (S', S") de 0,1 à 100 mm, de façon préférée de 0,1 à 50 mm, de façon particulièrement préférée de 0,1 à 15 mm et de façon tout particulièrement préférée de 0,1 à 5 mm.

5. Dispositif orthopédique (1) selon la revendication 3 ou 4, **caractérisé en ce que** l'interstice comporte une première dimension d'interstice (S') entre la surface intérieure du logement (2) et une première et une deuxième surface (30, 31) du corps de rallonge (3) et une deuxième dimension d'interstice (S") entre la surface intérieure du logement (2) et des surfaces latérales (32', 32") du corps de rallonge (3) et dans lequel la première et la deuxième dimension d'interstice sont différentes.

6. Dispositif orthopédique (1) selon l'une quelconque des revendications précédentes, **caractérisé en ce que** le dispositif comprend un moyen de rappel (5) du corps de rallonge (3) dans une position de départ dans le logement (2).

7. Dispositif orthopédique (1) selon la revendication 6, **caractérisé en ce que** le moyen de rappel (5) comprend une matière plastique élastique disposée entre le logement (2) et le corps de rallonge (3), un ressort, deux aimants permanents et/ou un joint.

8. Dispositif orthopédique (1) selon l'une quelconque des revendications précédentes, **caractérisé en ce que** les surfaces (30, 31, 32', 33") du corps de rallonge (3) et/ou du logement (2) reliées au fluide (4) dilaté et placées perpendiculairement à la normale de la direction de rallonge comprennent une structuration (33).

9. Dispositif orthopédique (1) selon la revendication 8, **caractérisé en ce que** la structuration (33) de la surface comprend des estampages, des nervures, des rainures, des picots et/ou une surface rugueuse.

10. Dispositif orthopédique (1) selon l'une quelconque des revendications précédentes, **caractérisé en ce que** le logement (2) comprend une ouverture (21) hors de laquelle le corps de rallonge (3) ressort.

11. Dispositif orthopédique (1) selon l'une quelconque des revendications précédentes, **caractérisé en ce que** le logement (2) comporte un joint (6) pour étanchéifier le mouvement du corps de rallonge (3).

12. Dispositif orthopédique (1) selon la revendication 11, **caractérisé en ce que** le joint (6) est relié, au niveau d'une première extrémité, au logement (2) et est relié, au niveau d'une deuxième extrémité, au corps de rallonge (3), le joint (6) pouvant être mobile à la façon d'un soufflet.

13. Dispositif orthopédique (1) selon l'une quelconque des revendications précédentes, **caractérisé en ce que** le logement et/ou le corps de rallonge sont fabriqués en matière plastique, de façon préférée en matière plastique thermoplastique, de façon particulièrement préférée en polypropylène, polyéthylène et/ou polyuréthane.

14. Dispositif orthopédique selon l'une quelconque des revendications précédentes, **caractérisé en ce que** le logement (2) et/ou le corps de rallonge (3) comprennent un dispositif de maintien (7) pouvant être placé au niveau d'une partie du corps.

15. Dispositif orthopédique selon l'une quelconque des revendications précédentes, **caractérisé en ce que** le fluide dilaté comprend une dispersion d'éthylène glycol, de dioxyde de silicium, de façon préférée du gel de silice avec une taille de particules de 5 à - 1000 nm et des surfaces de 30 à 250 m²/g, une partie de matière solide de 10 - 80 % en poids et des stabilisateurs.
